# EUROPÄISCHE PATENTSCHRIFT

(11) **EP 2 094 313 B1**
(45) Veröffentlichungstag und Bekanntmachung des Hinweises auf die Patenterteilung: **23.05.2012**
(21) Anmeldenummer: 07816233.6
(22) Anmeldetag: 08.11.2007
(51) Int. Cl.: A61L 2/08

(54) **ANLAGE ZUR STERILISATION VON OBJEKTEN MITTELS STRAHLUNGSQUELLE**
PLANT FOR STERILIZING OBJECTS BY MEANS OF A RADIATION SOURCE
INSTALLATION DE STÉRILISATION D'OBJETS AU MOYEN D'UNE SOURCE DE RAYONNEMENT

(30) Priorität: 08.11.2006 US 857569 P
(43) Veröffentlichungstag der Anmeldung: 02.09.2009
(73) Patentinhaber: SKAN AG, 4123 Allschwil (CH)
(72) Erfinder: NEWMAN, John Thomas, Thornton, CO 80029 (US); SIGWARTH, Volker, 4334 Sisseln (CH); SMITH, Paul Alexander, Los Alamos, MN 87544 (US)
(74) Vertreter: Ullrich, Gerhard
(86) Internationale Anmeldenummer: PCT/CH2007/000550
(87) Internationale Veröffentlichungsnummer: WO 2008/055375

(56) Entgegenhaltungen:
- EP-A- 0 570 946
- DE-A1- 3 321 195
- DE-A1- 3 911 749
- DE-B- 1 073 388
- US-A1- 2006 186 350

## Beschreibung

### Anwendungsgebiet der Erfindung

Die vorliegende Erfindung betrifft eine Anlage zur Sterilisation von Objekten mittels einer Strahlungsquelle zur Erzeugung von Röntgen-, Gamma- oder Elektronenbestrahlung, wie sie z.B. in der Industrie bei der Herstellung medizinischer Einwegartikel eingesetzt wird. Eine solche Anlage hat zunächst eine Bestrahlungszone, in der die Strahlungsquelle angeordnet ist. Der Bestrahlungszone ist eine Eingangszone vorgesetzt, der eine Beschickungszone zum Beladen der Anlage mit den zu behandelnden Objekten vorangeht. An die Bestrahlungszone schliesst sich eine Ausgangszone an, der eine Weiterbehandlungszone folgt, in welche die bestrahlten Objekte für den hier stattfindenden fortgesetzten Arbeitsprozess ausgegeben werden. Die Weiterbehandlungszone ist typisch als Isolator oder Reinraum beschaffen, in dem Personen tätig sein können. Zu behandelnde Objekte sind z.B. Behältnisse mit einer Vielzahl von zunächst leeren Injektionsspritzen, wobei die Behältnisse in der Anlage äusserlich zu sterilisieren sind und in der Weiterbehandlungszone befüllt werden. Durch die Anlage verläuft eine Transportstrecke, die zum Befördern der Objekte dient. Die Transportstrecke wird z.B. von einem Förderband gebildet. Zum Schutz der in der Umgebung der Anlage sich aufhaltenden Personen ist der Eingangszone und der Ausgangszone jeweils eine Abschirmung zugeordnet.

### Stand der Technik

Aus der Praxis sind Anlagen zur Strahlensterilisation bekannt, in denen die Transportstrecke in der Horizontalebene mäanderförmig ausgebildet ist. Die Strahlungsquelle positioniert man in einem hinter Kurven liegenden Abschnitt, wobei entlang der Transportstrecke Abschirmungen aufgestellt sind, welche die Strahlung mit jeder Umlenkung schwächen, so dass an der nach aussen offenen Beladungs- und Entladungsmündung der Transportstrecke keine für Personen gefährliche Strahlung mehr austritt.

In der WO 2006/111681 A2 ist eine Anlage zur Strahlensterilisation offenbart, bei der die Transportstrecke in der Vertikalebene mit jeweils einer Stufe an einem horizontal angeordneten Drehteller ausgebildet ist. Die Strahlungsquelle liegt zwischen den Drehtellern und wird so von diesen abgeschirmt. Die soweit bekannten Anlagen erfordern ein aufwendiges Transportsystem, das überdies durch den Bewegungsablauf nicht unwesentlichen Abrieb erzeugt, der als Verunreinigung in der Anlage beseitigt werden muss. Es entsteht die vermehrte Wahrscheinlichkeit, dass zu behandelnde Objekte auf der Transportstrecke nicht in der vorbestimmten Position verbleiben, sondern hängen bleiben oder herunterfallen. Schliesslich erfordern die kurven- oder stufenförmigen Transportstrecken mehr Zeit für den Durchlauf der zu behandelnden Objekte sowie einen erhöhten Platzbedarf für die gesamte Anlage.

Gegenstand der US 2006/0186350 A1 ist eine Anlage zur Sterilisation von Objekten mittels einer Strahlungsquelle. Die Anlage hat zunächst eine Bestrahlungszone, in der die Strahlungsquelle angeordnet ist. Der Bestrahlungszone ist eine Eingangszone vorgesetzt, der wiederum eine Beschickungszone vorangeht. An die Bestrahlungszone schliesst sich eine Ausgangszone an, der eine Weiterbehandlungszone folgt. Durch die Anlage verläuft eine Transportstrecke, die zum Befördern der Objekte dient. Zum Schutz des Bedienpersonals beim Einfahren der zu behandelnden Objekte in die Bestrahlungszone bzw. beim Ausschleusen der behandelten Objekte aus der Bestrahlungszone ist die Transportstrecke stufenförmig gestaltet, jeweils mit einem der Bestrahlungszone vor- und nachgeordneten Elevatorsystem.

### Aufgabe der Erfindung

Angesichts der Nachteile der bislang bekannten Anlagen, liegt der Erfindung die Aufgabe zugrunde, die Beförderung der in der Anlage zu behandelnden Objekte zu vereinfachen und zugleich die Strahlensicherheit strikt zu gewährleisten. Eine weitere Aufgabe besteht darin, eine qualifizierte Reinheitsklasse in der Anlage zumindest ab der Strahlungsquelle bis einschliesslich der Ausgangszone zu erreichen. Eine nächste Aufgabe ist, die Ummantelung der Anlage montage- und servicefreundlicher zu gestalten. Schliesslich besteht eine Aufgabe darin, die an der Strahlungsquelle erzeugten unerwünschten Gase, wie insbesondere Ozon, effizient aus der Anlage zu entfernen bzw. das Entstehen solcher Gase zumindest zu minimieren.

### Übersicht über die Erfindung

Die Anlage zur Sterilisation von Objekten mittels einer Strahlungsquelle hat zunächst eine Bestrahlungszone, in der die Strahlungsquelle angeordnet ist. Der Bestrahlungszone ist eine Eingangszone vorgesetzt, der wiederum eine Beschickungszone vorangeht. An die Bestrahlungszone schliesst sich eine Ausgangszone an, der eine Weiterbehandlungszone folgt. Durch die Anlage verläuft eine Transportstrecke, die zum Befördern der Objekte dient. Der Eingangszone und der Aus-gangszone ist jeweils eine Abschirmung zugeordnet. Die Eingangszone hat eine erste Eingangsöffnung mit Durchlass zur Beschickungszone und eine zweite Eingangsöffnung mit Durchlass zur Bestrahlungszone. Die Ausgangszone hat eine erste Ausgangsöffnung mit Durchlass zur Bestrahlungszone und eine zweite Ausgangsöffnung mit Durchlass zur Weiterbehandlungszone. Die Transportstrecke erstreckt sich fluchtend durch die erste Eingangsöffnung, die zweite Eingangsöffnung, die erste Ausgangsöffnung und die zweite Ausgangsöffnung. Die Abschirmungen sind beweglich und in jeder Stellungssituation der Abschirmungen, in welcher eine der beiden Eingangsöffnungen oder eine der beiden Ausgangsöffnungen die Passage der durch die Anlage beförderten Objekte erlaubt, ist die andere Eingangsöffnung sowie die andere Ausgangsöffnung von der jeweils zugeordneten Abschirmung überdeckt.

Die Abschirmung besteht aus zwei plattenförmigen, verschieblichen oder verschwenkbaren Elementen, zwischen denen sich eine Verbindung erstreckt. Die Elemente haben Materialbereiche, jedoch keine Aussparungen, und sind in einem Abstand angeordnet, welcher die dazwischen liegende Aufnahme eines Objektes erlaubt. Auch die Abschirmung dieser Alternative ist mit einem Antrieb verbunden.

Die Strahlungsquelle wirkt in einen Kanal hinein, in welchem die Transportstrecke zum Befördern der Objekte verläuft, und der sich einerseits zur zweiten Eingangsöffnung der Eingangszone und andererseits zur ersten Ausgangsöffnung der Ausgangszone erstreckt.

Das Gehäuse der Sterilisationsanlage ist aus aneinandergefügten, gegenseitig abgedichteten Sandwichelementen aufgebaut. Ein Sandwichelement besitzt zwischen zwei Aussenschichten, vorzugsweise aus Edelstahlblech, eine strahlungsabsorbierende innere Schicht, vorzugsweise aus Blei. Zwischen benachbart angeordneten Sandwichelementen ist eine Dichtung vorgesehen.

Der von der Strahlungsquelle beaufschlagte Behandlungsraum ist mit einem inerten Gas gefüllt, um das Entstehen unerwünschter Gase, wie insbesondere Ozon, zumindest zu minimieren.

### Kurzbeschreibung der beigefügten Zeichnun-gen

Es zeigen:
- Figur 1A -: den Aufbau einer Anlage zur Sterilisation von Objekten mittels einer Strahlungsquelle, mit anschliessender Weiterbehandlungszone, in Perspektivansicht;
- Figur 1B -: den Aufbau gemäss Figur 1A, in Frontansicht;

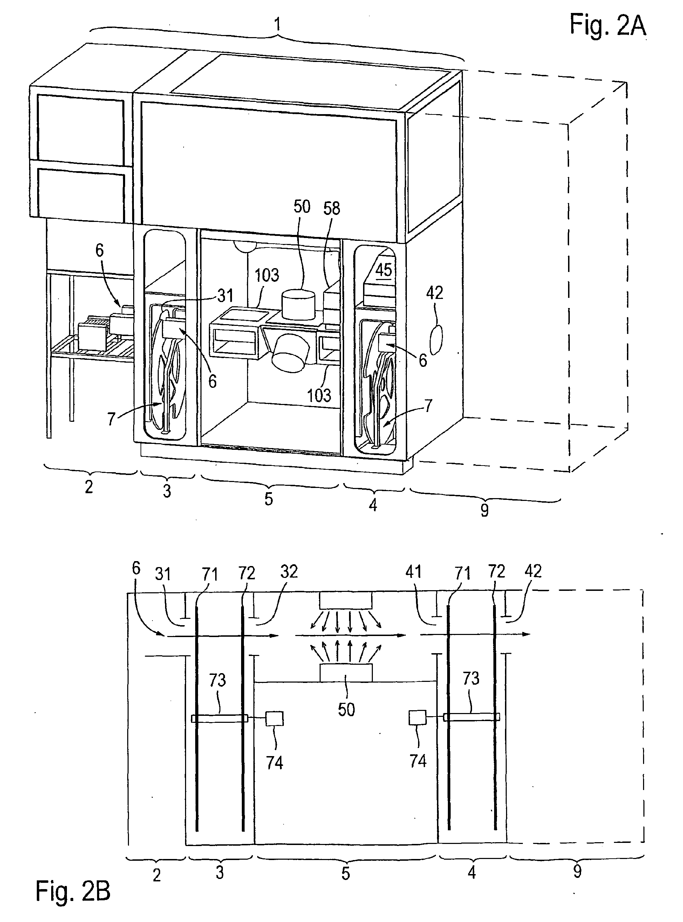

- Figur 2A -: den Aufbau gemäss Figur 1A, mit teilweiser Einsicht in das Innere der Anlage;
- Figur 2B -: den Aufbau gemäss Figur 1A, als Prinzipdarstellung;

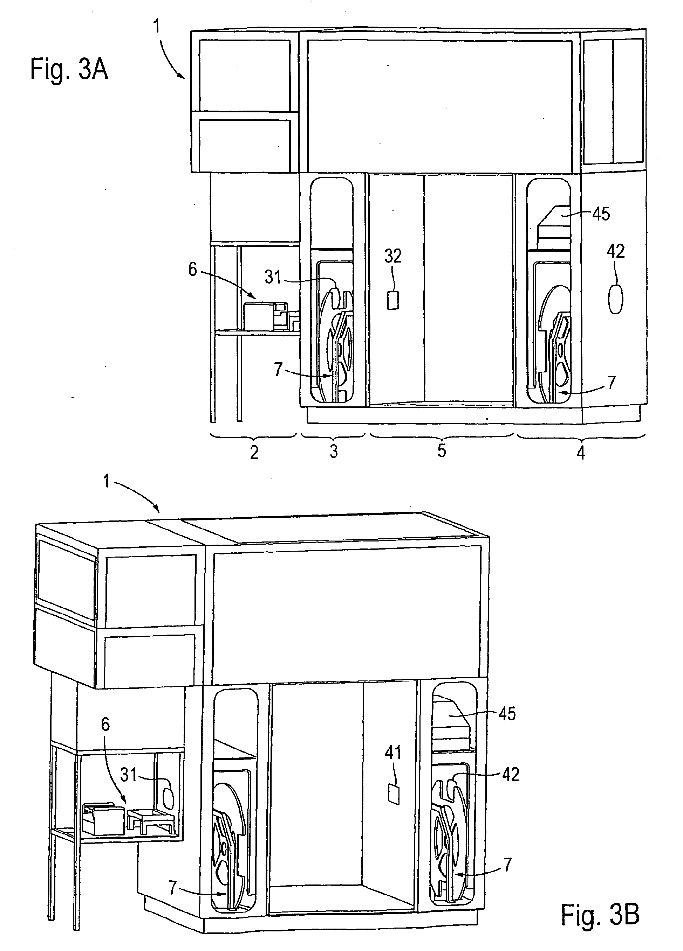

- Figur 3A -: den Aufbau gemäss Figur 1A, ohne Einbauten in der Bestrahlungszone und ohne Weiterbehandlungszone, in Perspektivansicht;
- Figur 3B -: den Aufbau gemäss Figur 3A, in gewechselter Perspektive;

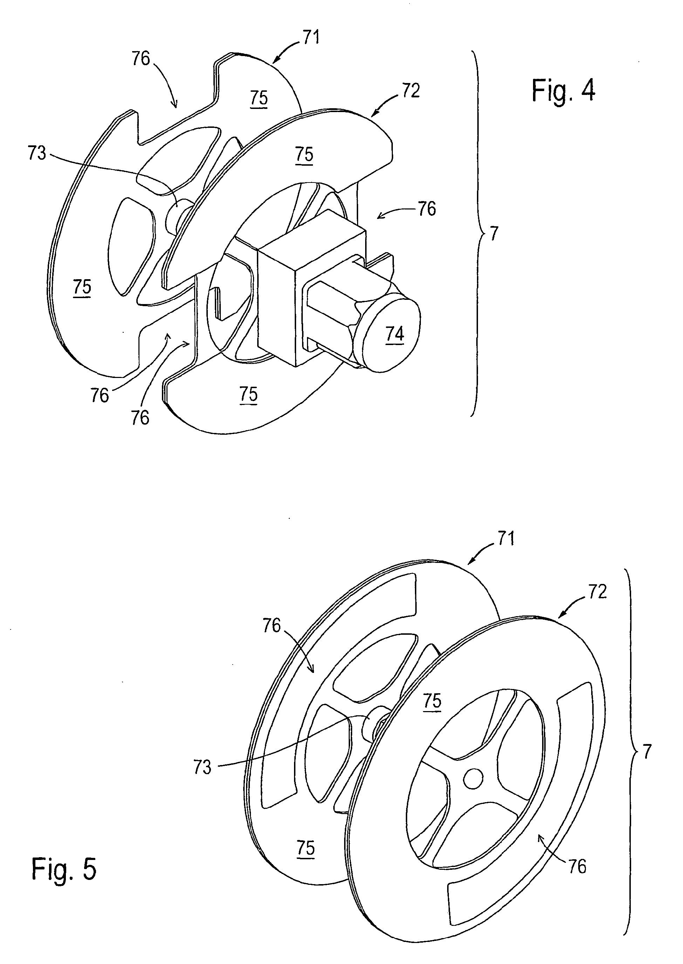

- Figur 4 -: eine Abschirmung aus Figur 2A, in einer *ersten Ausführung*, in Perspektivansicht;
- Figur 5 -: eine Abschirmung in einer *zvireiterAusführung*, in Perspektivansicht;

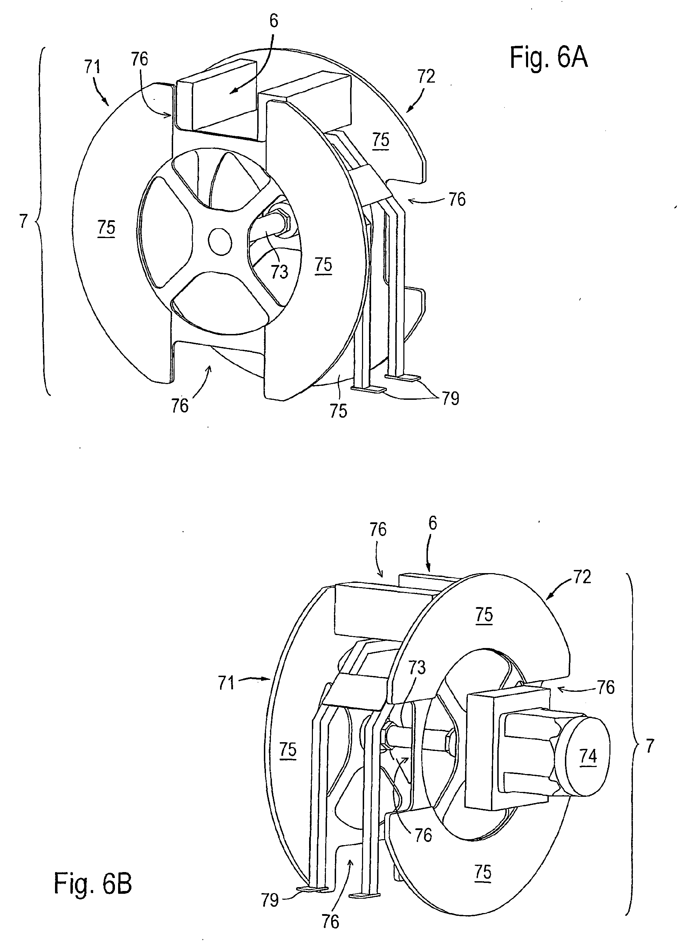

- Figur 6A -: die Abschirmung gemäss Figur 4, mit einem Abschnitt einer Transportstrecke und Gestell, in Perspektivansicht;
- Figur 6B -: den Aufbau gemäss Figur 6A, in gewechselter Perspektive;

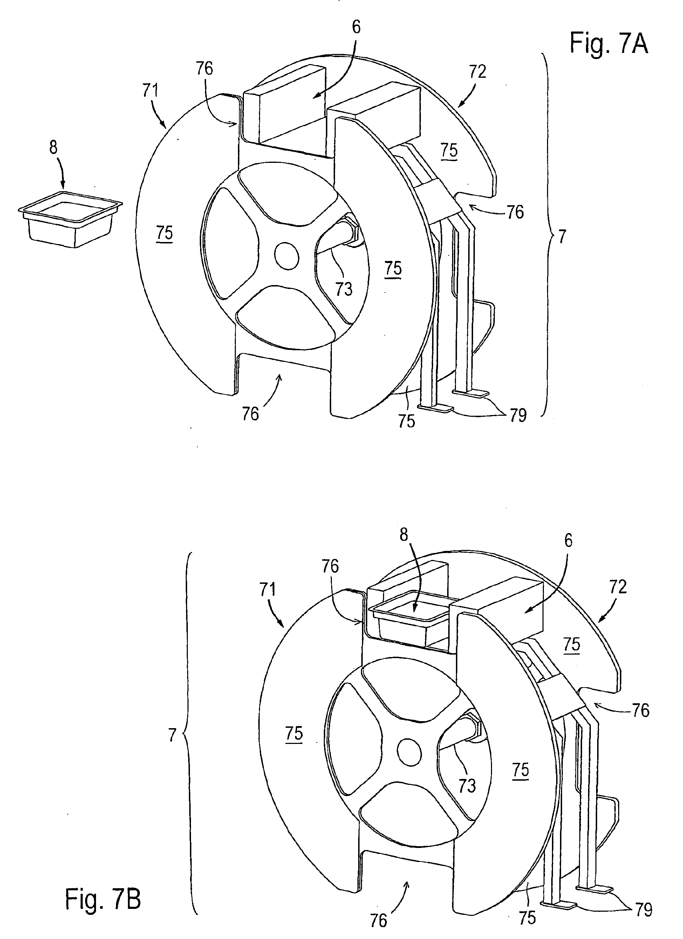

- Figur 7A -: den Aufbau gemäss Figur 6A, mit einem angenäherten zu behandelnden Objekt, in Perspektivansicht;
- Figur 7B -: den Aufbau gemäss Figur 7A, mit einem in die Abschirmung auf die Transportstrecke gebrachten Objekt, in Perspektivansicht;

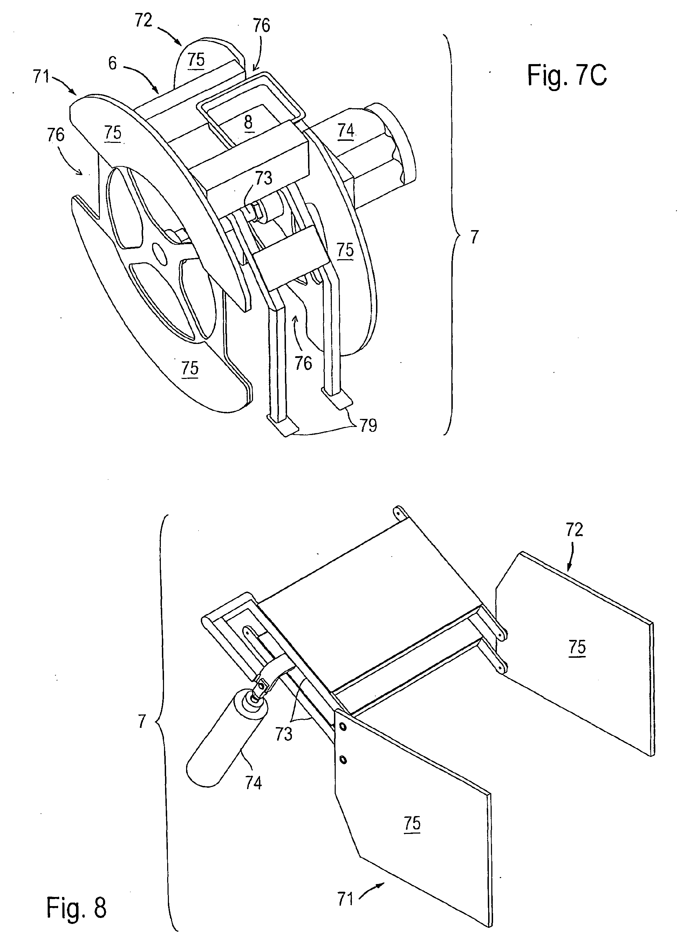

- Figur 7C -: den Aufbau gemäss Figur 7A, mit einem die Abschirmung über die Transportstrecke verlassenden Objekt, in Perspektivansicht;
- Figur 8 -: eine Abschirmung in einer *dritten Ausführung*, in Perspektivansicht;
- Figur 9A -: eine charakteristische Stellungssituation der Abschirmungen und zu behandelnden Objekte innerhalb der Anlage, als Prinzipdarstellung;
- Figur 9B -: eine weitere charakteristische Stellungssituation der Abschirmungen und zu behandelnden Objekte innerhalb der Anlage, als Prinzipdarstellung;
- Figur 10 -: zwei aneinander gefügte Sandwichelemente mit zwischen gelegter Dichtung, als Prinzipdarstellung; und

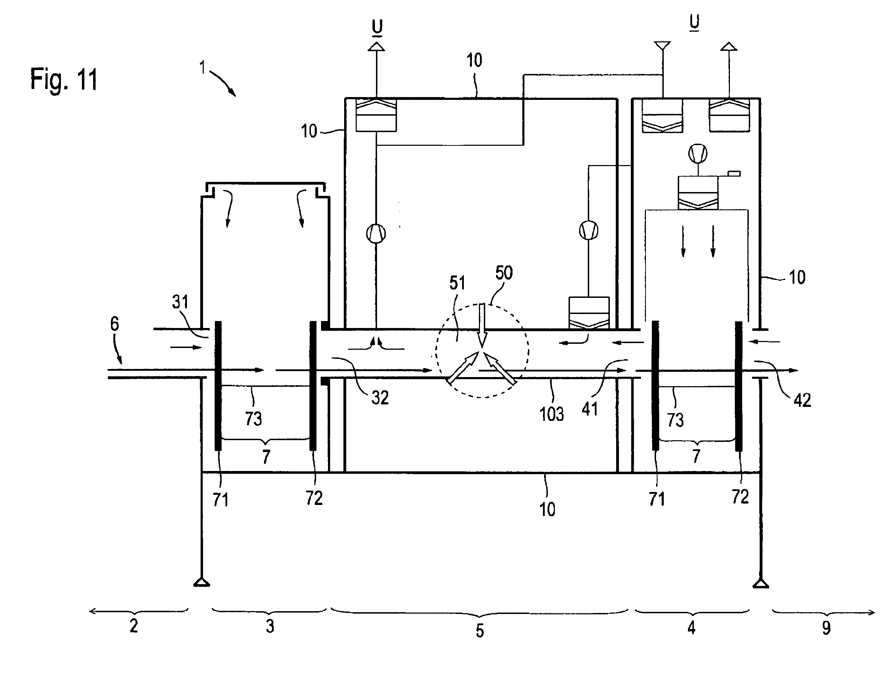

- Figur 11 -: die apparative Ausstattung der Anlage und darin stattfindender Strömungsverlauf als Prinzipdarstellung.

### Ausführungsbeispiel

Anhand der beiliegenden Zeichnungen erfolgt nachstehend die detaillierte Beschreibung eines Ausführungsbeispiels der Anlage zur Sterilisation von Objekten mittels einer Strahlungsquelle. Zur in der Anlage eingesetzten Abschirmung werden alternative Lösungen dargestellt und ferner - über die essentiellen Merkmale hinaus - vorteilhafte konstruktive Einzelheiten offenbart.

Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden Figurenbeschreibungen Bezug genommen. Im Interesse der Übersichtlichkeit wird auf die wiederholte Bezeichnung von Bauteilen in nachfolgenden Figuren zumeist verzichtet, sofern zeichnerisch eindeutig erkennbar ist, dass es sich um "wiederkehrende" Bauteile handelt.

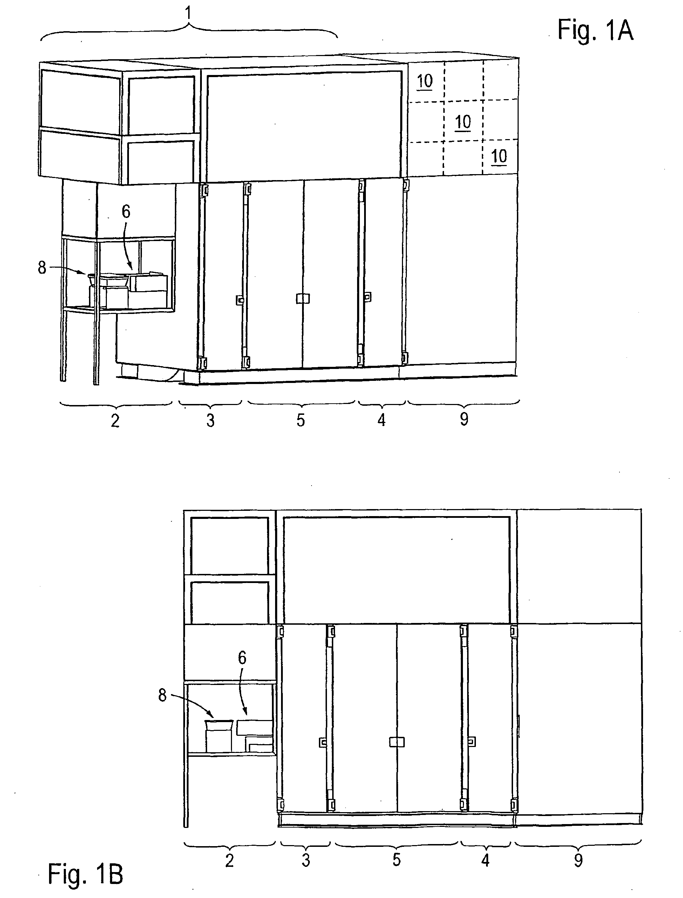

### Figuren 1A, 1 Bund 10

Das Zentrum der Anlage **1** bildet eine Bestrahlungszone **5,** der eine Eingangszone **3** vorgesetzt und eine Ausgangszone **4** nachgeordnet ist. Vor der Eingangszone **3** befindet sich eine Beschickungszone **2,** in welcher die Transportstrecke **6** zum Befördern der in der Anlage **1** zu behandelnden Objekte **8** beginnt. Hier wird die Anlage **1** mit den Objekten **8** beschickt und am Ende der Ausgangszone **4** werden die behandelten Objekte **8** ausgegeben, um in der Weiterbehandlungszone **9** dem fortgesetzten Arbeitsprozess unterzogen zu werden. An die Ausgangszone **4** schliesst sich eine Weiterbehandlungszone **9** an, typisch als Isolator oder Reinraum beschaffen, in welchem Personen tätig sein können. Solche Objekte **8** sind z.B. Behältnisse mit einer Vielzahl von zunächst leeren Injektionsspritzen, wobei die Behältnisse in der Anlage äusserlich zu sterilisieren sind und in der Weiterbehandlungszone **9** befüllt werden.

Das Gehäuse der Anlage **1** ist vorteilhaft aus einer Vielzahl von Sandwichelementen **10** zusammengesetzt. Ein Sandwichelement **10** hat innen eine dickere strahlungsabsorbierende Schicht **100** - üblich aus Blei -, die zwischen zwei Aussenschichten **101** liegt, welche z.B. aus Edelstahlblech bestehen. Zwischen aneinander grenzende Sandwichelemente **10** sind Dichtungen **102** eingesetzt.

### Figuren 2A bis 3B

Die Transportstrecke **6** beginnt in der Beschickungszone **2** und erstreckt sich fluchtend durch die gesamte Anlage **1,** also die Eingangszone **3,** die Bestrahlungszone **5** und die Ausgangszone **4.** Innerhalb der Bestrahlungszone **5** ist die Transportstrecke **6** von dem Kanal **103** umgeben, um welchen die Strahlungsquelle **50,** vorzugsweise in Gestalt eines Elektronenstrahlers, angeordnet ist. Die Strahlungsquelle **50** wirkt auf die den Kanal **103** auf der Transportstrecke **6** durchfahrende Objekte **8.** In der Eingangszone **3** und in der Ausgangszone **4** ist jeweils eine Abschirmung **7** eingebaut.

Die Eingangszone **3** hat eine erste Eingangsöffnung **31** mit Durchlass zur Beschickungszone **2** und eine zweite Eingangsöffnung **32** mit Durchlass zur Bestrahlungszone **5.** Die Ausgangszone **4** hat eine erste Ausgangsöffnung **41** mit Durchlass zur Bestrahlungszone **5** und eine zweite Ausgangsöffnung **42** mit Durchlass zur Weiterbehandlungszone **9.** Die Transportstrecke **6** erstreckt sich fluchtend durch die vier Öffnungen **31,32;41,42.** In einer ersten Ausführungsform hat die Abschirmung **7** zwei scheibenförmige, drehbare Elemente **71,72,** zwischen denen eine Verbindung **73,** ähnlich einer Achse, besteht. Auf die Abschirmung **7** wirkt ein Antrieb **74.** Die Elemente **71,72** erstrecken sich soweit über die vier Öffnungen **31,32;41,42** hinaus, dass in bestimmten Stellungssituationen diese Öffnungen **31,32;41,42** überdeckt werden. In der gezeigten Ausführung sind beide Elemente **71,72** innerhalb der Eingangszone **3** bzw. der Ausgangszone **4** positioniert. Alternativ könnte das erste Element **71** in der Beschickungszone **2** an der ersten Eingangsöffnung **31** und/oder das zweite Element **72** in der Bestrahlungszone **5** an der zweiten Eingangsöffnung **32** platziert sein.

### Figuren 4, 6A bis 7C, 9A und 9B

Die Abschirmung **7** hat zwei im Prinzip deckungsgleich parallel zueinander beabstandete, scheibenförmige Elemente **71,72,** zwischen denen eine axiale Verbindung **73** besteht. Jedes Element **71,72** hat an seinem Umfang zwei Aussparungen **76,** die radial offen und zueinander um 180° versetzt sind. Im übrigen Kreisring liegen Materialbereiche **75,** während zum Zentrum hin grossflächige Durchbrüche der Gewichtsersparnis dienen. Entsprechend der Funktion der Abschirmung **7** als Schutz gegen den von der Strahlungsquelle **50** emittierten Elektronenstrom, besitzen die Elemente **71,72** einen strahlungsabsorbierenden Aufbau mit einer Schicht aus Blei. Die Aussparungen **76** des ersten Elements **71** sind gegenüber den Aussparungen **76** des zweiten Elements **72** so weit versetzt, dass keine Überlappung auftritt. In der Spannweite müssen die Aussparungen **76** die Passage der Objekte **8** erlauben und der lichte Abstand zwischen den Elementen **71,72** muss so bemessen sein, um darin einen Abschnitt der Transportstrecke **6** und in letzterer ein Objekt **8** aufnehmen zu können. Zur Stützung der in die Anlage **1** eingebauten Abschirmung **7** dient ein Gestell **79.** Der mit der Abschirmung **7** gekoppelte Antrieb **74** ist vorzugsweise ein Elektromotor. Diese Ausführungsform der Abschirmung 7 ist für ein taktweises Weiterdrehen vorgesehen.

Bei einer Stellung der Abschirmung **7**, in welcher eine Aussparung **76** an der Passage der Transportstrecke **6** zu liegen kommt, lässt sich ein Objekt **8** zwischen die Elemente **71,72** einbringen oder andererseits entladen. Das Einbringen geschieht durch die erste Eingangöffnung **31** oder die erste Ausgangsöffnung **41.** Das Entladen erfolgt durch die zweite Ausgangöffnung **32** oder die zweite Ausgangsöffnung **42.** Steht hingegen ein Materialbereich **75** vor der Passage der Transportstrecke **6** - d.h. in der Anlage **1** vor einer der Öffnungen **31,32;41,42** - ist das Einbringen bzw. Entladen eines Objektes **8** blockiert, um den Strahlungsdurchlass zu verhindern.

Je nach Konzeption des Bearbeitungsdurchlaufs der zu behandelnden Objekte **8** durch die Anlage **1** wird man die Verstellung zwischen der in der Eingangszone **3** eingebauten Abschirmung **7** und der in der Ausgangszone **4** positionierten Abschirmung **7** koordinieren.

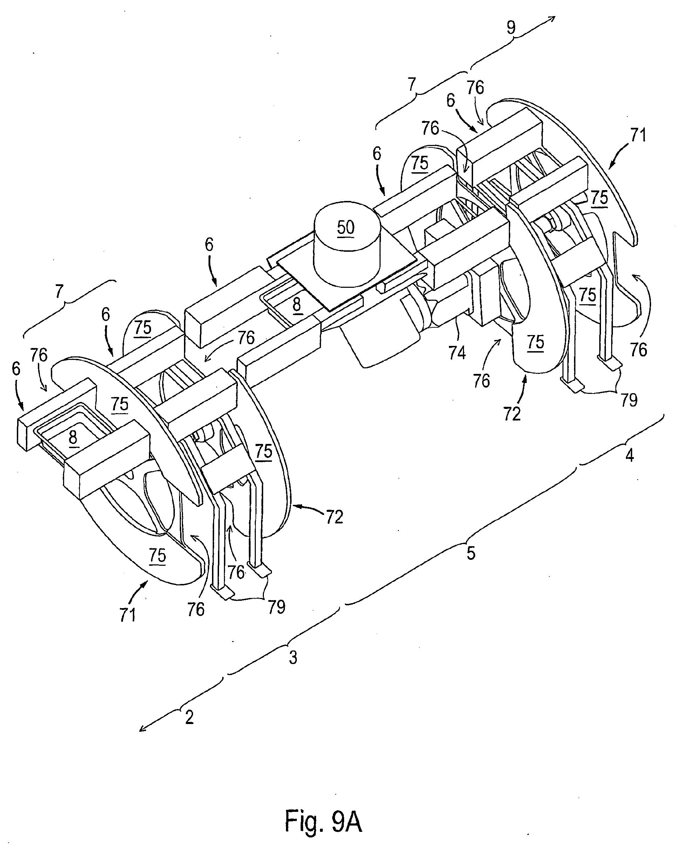

In der Stellungssituation gemäss Figur 9A sind:
- der Durchlass zwischen der Beschickungszone **2** und der Eingangszone **3** über die erste Eingangsöffnung **31** aufgrund des anstehenden Materialbereichs **75** gesperrt; ein Objekt **8** befindet sich vor der Eingangszone **3;**
- der Durchlass zwischen der Eingangszone **3** und der Bestrahlungszone **5** über die zweite Eingangsöffnung **32** aufgrund der anstehenden Aussparung **76** offen; ein Objekt **8** hat die Eingangszone **3** verlassen und durchfährt die Bestrahlungszone **5;**
- der Durchlass zwischen der Bestrahlungszone **5** und der Ausgangszone **4** über die erste Ausgangsöffnung **41** aufgrund der anstehenden Aussparung **76** offen; das Objekt **8** hat freie Einfahrt in die Ausgangszone **4;** und
- der Durchlass zwischen der Ausgangszone **4** und der Weiterbehandlungszone **9** über die zweite Ausgangsöffnung **42** aufgrund des anstehenden Materialbereichs **75** gesperrt.

Somit ist gesichert, dass in jeder Stellungssituation bei der vorteilhaft fluchtenden Transportstrecke **6,** mit den entsprechend angeordneten Öffnungen **31,32;41,42,** der Weg für die von der Strahlungsquelle **5** emittierte Strahlung in keiner Richtung entlang der Transportstrecke **6** niemals nach aussen frei ist. Eine Gefährdung von nahe der Anlage **1** sich aufhaltenden Personen ist damit ausgeschlossen.

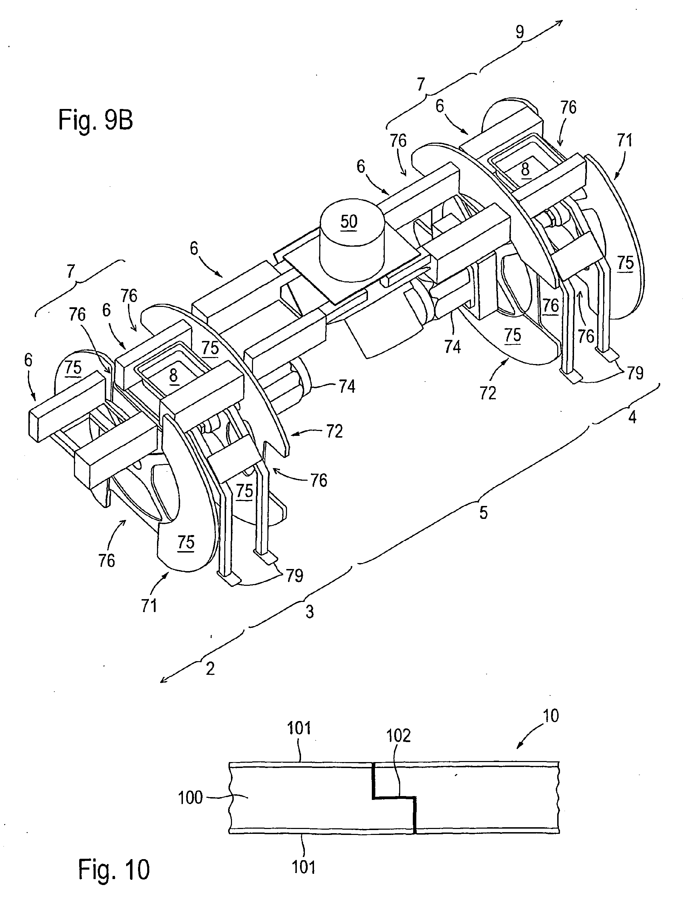

In der veränderten Stellungssituation gemäss Figur 9B sind:
- der Durchlass zwischen der Beschickungszone **2** und der Eingangszone **3** über die erste Eingangsöffnung **31** aufgrund der anstehenden Aussparung **76** offen; ein Objekt **8** ist von der Beschickungszone **2** in die Eingangszone **3** gefahren;
- der Durchlass zwischen der der Eingangszone **3** und der Bestrahlungszone **5** über die zweite Eingangsöffnung **32** aufgrund des anstehenden Materialbereichs **75** gesperrt; das Objekt **8** kann die Eingangszone **3** nicht verlassen;
- der Durchlass zwischen der Bestrahlungszone **5** und der Ausgangszone **4** über die erste Ausgangsöffnung **41** aufgrund des anstehenden Materialbereichs **75** gesperrt; und
- der Durchlass zwischen der Ausgangszone **4** und der Weiterbehandlungszone **9** über die zweite Ausgangsöffnung **42** aufgrund der anstehenden Aussparung **76** offen; das Objekt **8** kann die Ausgangszone **4** hin zur Weiterbehandlungszone **9** verlassen.

Auch hier ist gesichert, dass die emittierte Strahlung in keiner Richtung entlang der Transportstrecke **6** nach aussen austreten kann.

### Figur 5

Für ein kontinuierliches Rotieren der Abschirmung **7,** was die Nachteile des Stop and Go-Betriebs vermeidet, hat bei dieser alternativen Ausführungsform jedes Element **71,72** nur eine Aussparung **76,** welche im Kreisbogen ein Vielfaches der Grösse eines Objekts **8** beträgt und damit dessen Passage beim kontinuierlichen Bewegen der Abschirmung **7** erlaubt. Die Aussparungen **76** beider Elemente **71,72** sind nicht überlappend dimensioniert.

### Figur 8

Einen deutlich unterschiedlichen Aufbau hat die hiesige Ausführungsform der Abschirmung **7,** welche aus zwei plattenförmigen verschieblichen oder verschwenkbaren Elementen **71,72** besteht, zwischen denen sich eine Verbindung **73** zur koordinierten Bewegung beider Elemente **71,72** erstreckt. Auch diese Abschirmung **7** ist mit einem Antrieb **74** verbunden, jedoch dehnt sich der Materialbereich **75** über jedes Element **71,72** aus, ohne dass daran Aussparungen **76** vorhanden sind.

### Figur 11

Die Strahlungsquelle **50** wirkt in einen Kanal **103** hinein, in welchem die Transportstrecke **6** zum Befördern der Objekte **8** verläuft, und der sich einerseits zur zweiten Eingangsöffnung **32** der Eingangszone **3** und andererseits zur ersten Ausgangsöffnung **41** der Ausgangszone **4** erstreckt. Zur Erzielung einer qualifizierten Reinheitsklasse von der Strahlungsquelle **50** bis über die gesamte Ausgangszone **4** wird letztere mit unidirektionaler Luftströmung betrieben.

Zur Vermeidung, insbesondere von z.B. Ozon, das bei eingeschalteter Strahlungsquelle **50** durch Reaktion mit der betroffenen Luft entsteht, ist der von der Strahlungsquelle **50** beaufschlagte Behandlungsraum innerhalb des Kanals **103** mit einem Inertgas **51** gefüllt.

## Patentansprüche

1. Anlage (**1**) zur Sterilisation von Objekten (**8**) mittels einer Strahlungsquelle (**50**), mit:
a) einer Bestrahlungszone (**5**), in der die Strahlungsquelle (**50**) angeordnet ist;
b) einer Eingangszone (**3**), die der Bestrahlungszone (**5**) vorgesetzt ist;
c) einer Beschickungszone (**2**), die der Eingangszone (**3**) vorgesetzt ist;
d) einer Ausgangszone (**4**), die sich an die Bestrahlungszone (**5**) anschliesst und der eine Weiterbehandlungszone (**9**) folgt;
e) einer Transportstrecke (**6**), die durch die Anlage (**1**) verläuft und zum Befördern der Objekte (**8**) dient;
f) einer Abschirmung (**7**), die der Eingangszone (**3**) zugeordnet ist; und
g) einer Abschirmung (**7**), die der Ausgangszone (**4**) zugeordnet ist, **dadurch gekennzeichnet, dass**
h) die Eingangszone (**3**) eine erste Eingangsöffnung (**31**) mit Durchlass zur Beschickungszone (**2**) und eine zweite Eingangsöffnung (**32**) mit Durchlass zur Bestrahlungszone (**5**) hat;
i) die Ausgangszone (**4**) eine erste Ausgangsöffnung (**41**) mit Durchlass zur Bestrahlungszone (**5**) und eine zweite Ausgangsöffnung (**42**) mit Durchlass zur Weiterbehandlungszone (**9**) hat;
j) die Transportstrecke (**6**) sich fluchtend durch die erste Eingangsöffnung (**31**), die zweite Eingangsöffnung (**32**), die erste Ausgangsöffnung (**41**) und die zweite Ausgangsöffnung (**42**) erstreckt;
k) die Abschirmungen (**7**) beweglich sind und in jeder Stellungssituation der Abschirmungen (**7**), in welcher eine der beiden Eingangsöffnungen (**31,32**) oder eine der beiden Ausgangsöffnungen (**41,42**) die Passage der durch die Anlage (**1**) beförderten Objekte (**8**) erlaubt, die andere Eingangsöffnung (**32,31**) sowie die andere Ausgangsöffnung (**42,41**) von der jeweils zugeordneten Abschirmung (**7**) überdeckt ist;
l) eine Abschirmung (**7**) aus zwei plattenförmigen, verschieblichen oder verschwenkbaren Elementen (**71,72**) besteht, zwischen denen sich eine Verbindung (**73**) erstreckt;
m) die Elemente (**71,72**) Materialbereiche (**75**) haben;
n) die Elemente. (**71,72**) in einem Abstand angeordnet sind, welcher die dazwischen liegende Aufnahme eines Objektes (**8**) erlaubt; und
o) die Abschirmung (**7**) mit einem Antrieb (**74**) verbunden ist.

2. Anlage (**1**) nach Anspruch 1, **dadurch gekennzeichnet, dass**
die Strahlungsquelle (**50**) in einen Kanal (**103**) hineinwirkt, in welchem die Transportstrecke (**6**) zum Befördern der Objekte (**8**) verläuft, und der sich einerseits zur zweiten Eingangsöffnung (**32**) der Eingangszone (**3**) und andererseits zur ersten Ausgangsöffnung (**41**) der Ausgangszone (**4**) erstreckt.

3. Anlage (1) nach Anspruch 1 oder 2, **dadurch gekennzeichnet, dass**
a) das Gehäuse der Sterilisationsanlage (**1**) aus aneinandergefügten, gegenseitig abgedichteten Sandwichelementen (**10**) aufgebaut ist;
b) ein Sandwichelement (**10**) zwischen zwei Aussenschichten (**101**), vorzugsweise Edelstahlblech, eine strahlungsabsorbierende innere Schicht (**100**), vorzugsweise aus Blei, besitzt; und
c) zwischen benachbart angeordneten Sandwichelementen (**10**) eine Dichtung (**102**) vorgesehen ist.

4. Anlage (**1**) nach einem der Ansprüche 1 bis 3, **dadurch gekennzeichnet, dass** der von der Strahlungsquelle (**50**) beaufschlagte Behandlungsraum eine Füllung von Inertgas enthält.

## Claims

1. Installation (**1**) for sterilizing objects (**8**) by means of a radiation source (**50**), with:
a) an irradiation zone (**5**) in which the radiation source (**50**) is arranged;
b) an entry zone (**3**), which is in front of the irradiation zone (**5**);
c) a feed zone (**2**), which is in front of the entry zone (**3**);
d) an exit zone (**4**), which adjoins the irradiation zone (**5**) and which is followed by a subsequent processing zone (**9**);
e) a transportation line (**6**) which passes through the installation (**1**) and is used to convey the objects (**8**);
f) a shield (**7**) which is assigned to the entry zone (**3**); and
g) a shield (**7**) which is assigned to the exit zone (**4**), **characterised in that**
h) the entry zone (**3**) has a first inlet opening (**31**) with a passage to the feed zone (**2**) and a second inlet opening (**32**) with a passage to the irradiation zone (**5**);
i) the exit zone (**4**) has a first outlet opening (**41**) with a passage to the irradiation zone (**5**) and a second outlet opening (**42**) with a passage to the subsequent processing zone (**9**);
j) the transportation line (**6**) extends flush through the first inlet opening (**31**), the second inlet opening (**32**), the first outlet opening (**41**) and the second outlet opening (**42**);
k) the shields (**7**) are movable and in every situation with regard to position of the shields (**7**) in which one of the two inlet openings (**31,32**) or one of the two outlet openings (**41,42**) permits the passage of the objects (**8**) transported through the installation (**1**), the other inlet opening (**32,31**) and the other outlet opening (**42,41**) are covered by the respectively assigned shield (**7**);
l) one shield (**7**) is composed of two plate-shaped, displaceable or pivotable elements (**71,72**), a connection (**73**) extending between the two;
m) the elements (**71,72**) have material regions (**75**);
n) the elements (**71, 72**) are arranged at a distance which allows an object (**8**) to be held between the former; and
o) the shield (**7**) is connected to a drive (**74**).

2. Installation (**1**) according to Claim 1, **characterised in that** the radiation source (**50**) acts into a channel (**103**) through which the transportation line (**6**) for conveying the objects (**8**) passes and which firstly extends to the second inlet opening (**32**) of the entry zone (**3**) and secondly extends to the first outlet opening (**41**) of the exit zone (**4**).

3. Installation (**1**) according to Claim 1 or 2, **characterised in that**
a) the housing of the sterilizing installation (**1**) is manufactured from sandwich elements (**10**) which adjoin one another and are sealed off against one another;
b) a sandwich element (**10**) has a radiation absorbing inner layer (**100**), preferably composed of lead, between two outer layers (**101**), preferably composed of a stainless steel plate; and
c) a seal (**102**) is provided between adjacently arranged sandwich elements (**10**).

4. Installation (**1**) according to one of Claims 1 to 3, **characterised in that** the treatment space acted upon by the radiation source (**50**) contains a filling of inert gas.

## Revendications

1. Installation (**1**) de stérilisation d'objets (**8**) au moyen d'une source de rayonnement (**50**), comportant:
a) une zone de rayonnement (**5**), dans laquelle la source de rayonnement (**50**) est disposée;
b) une zone d'entrée (**3**), qui est disposée en amont de la zone de rayonnement (**5**);
c) une zone de chargement (**2**), qui est disposée en amont de la zone d'entrée (**3**);
d) une zone de sortie (**4**), qui se raccorde à la zone de rayonnement (**5**) et à laquelle succède une zone de post-traitement (**9**);
e) une section de transport (**6**), qui s'étend à travers l'installation (**1**) et est destinée au transport des objets (**8**);
f) un écran (**7**), qui est associé à la zone d'entrée (**3**); et
g) un écran (**7**), qui est associé à la zone de sortie (**4**), **caractérisée en ce que**
h) la zone d'entrée (**3**) présente une première ouverture d'entrée (**31**) avec passage vers la zone de chargement (**2**) et une deuxième ouverture d'entrée (**32**) avec passage vers la zone de rayonnement (**5**);
i) la zone de sortie (**4**) présente une première ouverture de sortie (**41**) avec passage vers la zone de rayonnement (**5**) et une deuxième ouverture de sortie (**42**) avec passage vers la zone de post-traitement (**9**);
j) la section de transport (**6**) s'étend en alignement à travers la première ouverture d'entrée (**31**), la deuxième ouverture d'entrée (**32**), la première ouverture de sortie (**41**) et la deuxième ouverture de sortie (**42**);
k) les écrans (**7**) sont mobiles et, dans chaque situation de positionnement des écrans (**7**), dans laquelle une des deux ouvertures d'entrée (**31,32**) ou une des deux ouvertures de sortie (**41,42**) permet le passage des objets (**8**) transportés à travers l'installation (**1**), l'autre ouverture d'entrée (**32,31**) ainsi que l'autre ouverture de sortie (**42,41**) est recouverte par l'écran (**7**) respectivement associé;
l) un écran (**7**) se compose de deux éléments en forme de plaques (**71,72**), aptes à coulisser ou à pivoter, entre lesquels s'étend une liaison (**73**);
m) les éléments (**71,72**) comportent des zones de matière (**75**);
n) les éléments (**71,72**) sont situés à une distance, qui permet la réception entre eux d'un objet (**8**); et
o) l'écran (**7**) est relié à un entraînement (**74**).

2. Installation (**1**) selon la revendication 1, **caractérisée en ce que** la source de rayonnement (**50**) exerce son action dans un canal (**103**), dans lequel s'étend la section de transport (**6**) destinée au transport des objets (**8**), et qui s'étend d'une part jusqu'à la deuxième ouverture d'entrée (**32**) de la zone d'entrée (**3**) et d'autre part jusqu'à la première ouverture de sortie (**41**) de la zone de sortie (**4**).

3. Installation (**1**) selon la revendication 1 ou 2, **caractérisée en ce que**
a) l'enceinte de l'installation de stérilisation (**1**) est composée d'éléments sandwich (**10**) assemblés les uns aux autres et étanches les uns par rapport aux autres;
b) un élément sandwich (**10**) possède entre deux couches extérieures (**101**), de préférence en tôle d'acier allié, une couche intérieure absorbant le rayonnement (**100**), de préférence en plomb; et
c) il est prévu un joint d'étanchéité (**102**) entre des éléments sandwich (**10**) disposés à côté l'un de l'autre.

4. Installation (**1**) selon l'une quelconque des revendications 1 à 3, **caractérisée en ce que** la chambre de traitement exposée à la source de rayonnement (**50**) contient une atmosphère de gaz inerte.
